# EUROPÄISCHE PATENTSCHRIFT

(11) **EP 1 487 816 B1**
(45) Veröffentlichungstag und Bekanntmachung des Hinweises auf die Patenterteilung: **16.01.2008**
(21) Anmeldenummer: 03706517.4
(22) Anmeldetag: 17.02.2003
(51) Int. Cl.: C07D 321/00, C11B 9/00

(54) **ETHERLACTON**
ETHER LACTONE
LACTONE ETHER

(30) Priorität: 28.03.2002 DE 10213899
(43) Veröffentlichungstag der Anmeldung: 22.12.2004
(73) Patentinhaber: KAO CORPORATION, Tokyo 103-8210 (JP)
(72) Erfinder: MARKERT, Thomas, 40789 Monheim (DE); WESTFECHTEL, Alfred, 40724 Hilden (DE); PORRMANN, Volker, 40723 Hilden (DE)
(74) Vertreter: HOFFMANN EITLE
(86) Internationale Anmeldenummer: PCT/EP2003/001562
(87) Internationale Veröffentlichungsnummer: WO 2003/082851

(56) Entgegenhaltungen:
- R. H. WRIGHT: "The Musk Odour" PERFUMERY AND ESSENTIAL OIL RECORD, Bd. 58, Nr. 9, 1967, Seiten 648-650, XP008017465 in der Anmeldung erwähnt

## Beschreibung

### Gebiet der Erfindung

Die vorliegende Erfindung betrifft ein Etherlacton spezieller Struktur sowie dessen Verwendung als Riechstoff.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5000 kg Rosenblüten notwendig. Die Folgen sind eine sehr stark limitierte Weltjahresproduktion sowie ein hoher Preis. Es ist daher klar, dass die Riechstoffindustrie einen ständigen Bedarf an neuen Riechstoffen mit interessanten Duftnoten hat. Einerseits kann dadurch die Palette der natürlich verfügbaren Riechstoffe ergänzt werden, andererseits ist es dadurch möglich, die notwendigen Anpassungen an wechselnde modische Geschmacksrichtungen vornehmen zu können. Darüber hinaus wird es auf diese Weise möglich, den ständig steigenden Bedarf an Geruchsverbesserern für Produkte des täglichen Bedarfs wie Kosmetika und Reinigungsmittel decken zu können.

Im Übrigen besteht generell ein ständiger Bedarf an synthetischen Riechstoffen, die sich günstig und mit gleichbleibend hoher Qualität herstellen lassen und die originelle olfaktorische Eigenschaften haben. Insbesondere sollen sie angenehme, möglichst naturnahe und qualitativ neuartige Geruchsprofile von ausreichender Intensität besitzen und in der Lage sein, den Duft von kosmetischen und Verbrauchsgütern vorteilhaft zu beeinflussen. Mit anderen Worten: es besteht ein ständiger Bedarf an Verbindungen, die charakteristische neue Geruchsprofile bei gleichzeitig hoher Haftfestigkeit, Geruchsintensität und Strahlkraft aufweisen.

In Perfum. Essent. Oil Rec. 1967, 58(9), Seiten 648-650 wird in Figure 1 auf Seite 648 in Spalte 5, Zeile 5 der Tabelle ein formelmäßig näher umschriebenes Etherlacton aufgeführt, das einen "mittleren" Moschusgeruch aufweist.

### Beschreibung der Erfindung

Es wurde gefunden, dass das Etherlacton der allgemeinen Formel (I) die oben genannten Forderungen in jeder Hinsicht ausgezeichnet erfüllen und in vorteilhafter Weise als Riechstoff mit unterschiedlichen nuancierten Geruchsnoten mit guter Haftfestigkeit eingesetzt werden kann.

Gegenstand der vorliegenden Erfindung ist zunächst das Etherlacton der allgemeinen Struktur (I)

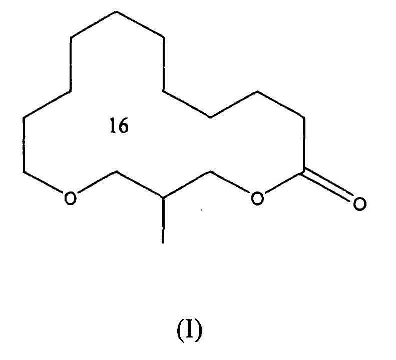

Die Erfindung betrifft ferner die Verwendung des Etherlactons der oben näher bezeichneten allgemeinen Struktur (I) als Riechstoff.

Die Verbindung (I) zeichnet sich durch eine Geruchscharakteristik aus, in der der Moschus-Charakter dominiert. Neben einer intensiven Moschusnote treten Duftaspekte hervor, die als süßlich, balsamisch, mit Wachsnoten beschrieben werden können. (I) weist eine ausgezeichnete Stabilität in Rezepturen der Kosmetik und Gebrauchsparfümerie auf.

Die Herstellung der Verbindung (I) kann nach bekannten Syntheseverfahren der organischen Chemie erfolgen.

In Parfüm-Kompositionen verstärkt die Verbindung (I) die Harmonie und die Ausstrahlung sowie die Natürlichkeit und auch die Haftung, wobei die Dosierung unter Berücksichtigung der übrigen Bestandteile der Komposition auf die jeweils angestrebte Duftnote abgestimmt wird.

Dass die Verbindung (I) die oben genannten Duftnoten aufweist, war nicht vorhersehbar und ist damit eine weitere Bestätigung für die allgemeine Erfahrung, dass die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluss der chemischen Struktur auf die Duftwahrnehmung hinreichend erforscht sind und somit also normalerweise nicht vorhergesehen werden kann, ob ein geänderter Aufbau bekannter Riechstoffe überhaupt zur Änderung der olfaktorischen Eigenschaften führt und ob diese Änderungen vom Fachmann positiv oder negativ beurteilt werden.

Es sei ausdrücklich darauf hingewiesen, daß sich die Duftcharakteristik der erfindungsgemäße Verbindung (I) von dem in Perfum. Essent. Oil Rec. 1967, 58(9), Seiten 648-650 in Figure 1 auf Seite 648 in Spalte 5, Zeile 5 der Tabelle formelmäßig näher umschriebenen Etherlacton mit lediglich "mittlerem" Moschusgeruch klar unterscheidet. Für den Fachmann war also nicht vorhersehbar oder naheliegend, ausgehend von der in Perfum. Essent. Oil Rec. 1967, 58(9), Seiten 648-650 in Figure 1 auf Seite 648 in Spalte 5, Zeile 5 genannten Struktur durch Einführung eines Methyl-Substituenten an einer ganz bestimmten Stelle zu einer Verbindung zu gelangen, die einen deutlich intensiveren Moschus-Geruch mit zusätzlichen weiteren Duftnoten aufweist.

Die Verbindung der Formel (I) eignet sich aufgrund ihres Geruchsprofils insbesondere auch zur Modifizierung und Verstärkung bekannter Kompositionen. Hervorgehoben werden soll insbesondere ihre außerordentliche Geruchsstärke, die ganz allgemein zur Veredelung der Komposition beiträgt.

Die Verbindung der Formel (I) läßt sich mit zahlreichen bekannten Riechstoffingredienzien, beispielsweise anderen Riechstoffen natürlichen, synthetischen oder partial-synthetischen Ursprungs, ätherischen Ölen und Pflanzenextrakten kombinieren. Die Palette der natürlichen Riechstoffe kann dabei sowohl leicht-, als auch mittel- und schwerflüchtige Komponenten umfassen. Die Palette der synthetischen Riechstoffe kann Vertreter aus praktisch allen Stoffklassen umfassen.

Beispiele für geeignete Substanzen, mit denen die Verbindung (I) kombiniert werden kann sind insbesondere:
(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl, Myrrheöl, Olibanumöl, Cedernholzöl, Sandelholzöl, ostindisch, Guajakholzöl, Cabreuva,
(b) Alkohole wie Farnesol, Geraniol, Citronellol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol], Mugetanol,
(c) Aldehyde wie Citral, Helional^{R}, alpha-Hexylzimtaldehyd, Hydroxycitronellal, Lilial^{R} [p-tert.-Butyl-α-methyldihydrozimtaldehyd], Methylnonylacetaldehyd,
(d) Ketone wie Allylionon, α-Ionon, β-Ionon, Isoraldein, Methylionon, Nootkaton, Calone, α-, β- und γ-Irone, Damascone,
(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat, Isobornylisobutyrat, Evernyl,
(f) Lactone wie gamma-Undecalacton, 1-Oxaspiro[4.4]nonan-2-on, Cylopentadecanolid, Ethylenbrassylat,
g) Ether wie Herbavert, Ambroxan,
sowie verschiedene weitere in der Parfümerie oft benutzte Komponenten wie Moschus- und Sandelholz-Riechstoffe, Indol, p-Menthan-8-thiol-3-on, Methyleugenol und Methylanthranilat.

Bemerkenswert ist ferner die Art und Weise, wie die Verbindung der Struktur (I) die Geruchsnoten einer breiten Palette bekannter Kompositionen abrundet und harmonisiert, ohne jedoch in unangenehmer Weise zu dominieren.

Die einsetzbaren Anteile der Verbindung (I) in Riechstoffkompositionen bewegen sich von etwa 1-70 Gew. %, bezogen auf die gesamte Mischung. Verbindung (I) sowie Riechstoffkompositionen, die die Verbindung (I) enthalten, können sowohl zur Parfümierung kosmetischer Präparate wie Lotionen, Cremes, Shampoos, Seifen, Salben, Pudern, Aerosolen, Zahnpasten, Mundwässern, Deodorantien als auch in der alkoholischen Parfümerie (z.B. Eau de Cologne, Eau de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,01 - 2 Gew. % - bezogen auf das gesamte Produkt - zugesetzt. Diese Werte sind jedoch keine beschränkenden Grenzwerte, da der erfahrenen Parfümeur auch mit noch geringeren Konzentrationen noch Effekte erzielen oder mit noch höheren Dosierungen neuartige Komplexe aufbauen kann.

### Beispiele

### Beispiel 1:

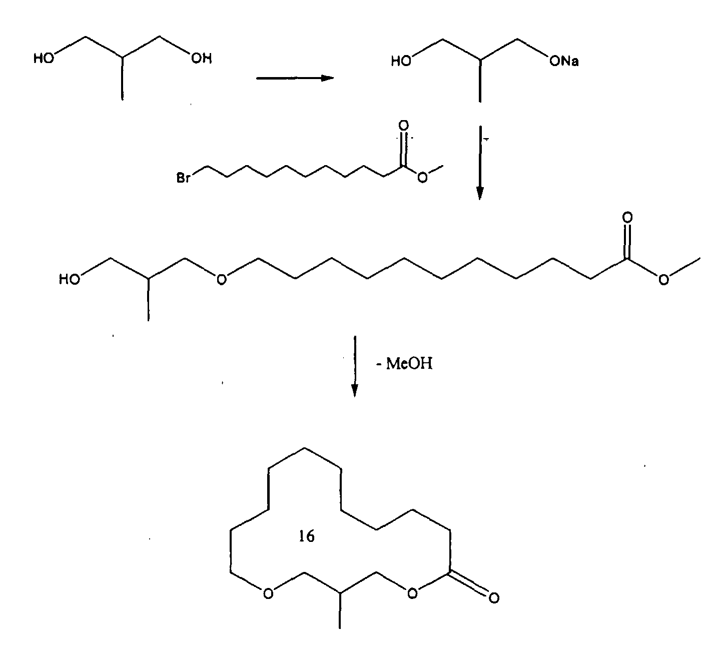

90 g (1 mol) 2-Methylpropan-1,3-diol und 27 g (0,5 mol) Natriummethylat wurden in 200 ml Xylol suspendiert und am Wasserabscheider bei 140 °C unter starkem Rühren 24 Stunden zum Rückfluß erhitzt. Anschließend wurden bei 140 °C 99 g (0,35 mol) 11-Bromundecansäuremethylester in ca. 3 Stunden kontinuierlich zu der Mischung zugetropft und weitere 3 Stunden am Rückfluß gekocht. Danach zeigte das Gaschromatogramm noch ca. 25 % unumgesetzten Bromundecansäuremethylester. Es wurden 90 g Natriummethylat 30%ig in Methanol und 10 g Polyethylenglykol 600 zugefügt und weitere 4 Stunden bei 132 °C am Wasserabscheider erhitzt, wobei Methanol abdestilliert wurde.

### Aufarbeitung:

Das Reaktionsgemisch wurde eingeengt, die dabei erhaltenen 80 g Rohprodukt wurden an einer Kugelrohrdestille langsam überdestilliert. Es wurden 20 g 30%iges Produkt und 20,2 g 75%iges Material in Fraktionen aufgefangen.

### Analytik:

Das IR-Spektrum (ATR-Technik) zeigte charakteristische Banden bei 1112, 1174, 1248, 1733 cm⁻¹ und einen CH-Schwingungsbereich von 2858 bis 2929 cm⁻¹.

Das ¹H-NMR-Spektrum (in CDCl₃, 400 MHz) zeigte 1 Methylgruppe (2 Singuletts, 3H) bei 0,95 ppm. Ein Signalberg bei. 1,3 ppm entspricht 5CH₂-Gruppen, 2 breite Signale bei 1,6 und 1,7 ppm 3 CH₂-Gruppen und ein Signal bei 2,3 ppm 1 CH₂-Gruppe. Das Signal bei 2,0 ppm wird dem CH, an dem die Methylgruppe sitzt zugeordnet. Ein komplex aufgespaltenes Signalgemisch lag im Bereich von 3,3 bis 4,2 ppm; dies entspricht 3 CH₂-Gruppen in Nachbarschaft zu den Sauerstoffatomen im Ring.

### Geruchsbeschreibung:

Im Angeruch: typisch Moschus, süßlich, balsamisch mit Wachs- und Noten wie heißes Bügeleisen; im Nachgeruch, nach 24 Stunden am Riechstreifen: Moschus, warmes Wachs, dieser Geruch haftet einige Wochen lang.

## Patentansprüche

1. Etherlacton der allgemeinen Struktur (I)

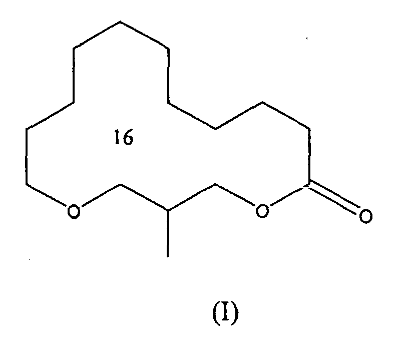

2. Verwendung des Etherlactons der allgemeinen Struktur (I) 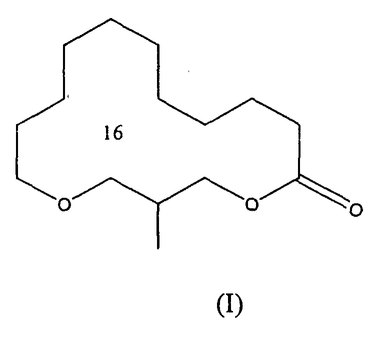 als Riechstoff.

3. Riechstoff-Kompositionen mit einem Gehalt an dem Etherlacton (I) 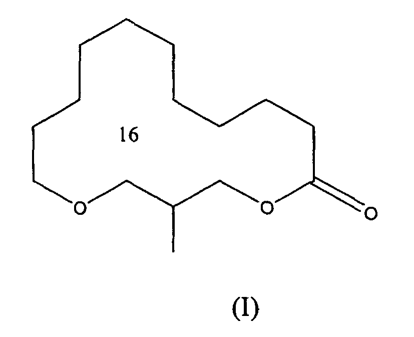 wobei die Verbindung (I) in einer Menge von 1 bis 70 Gew.-% - bezogen auf die gesamte Komposition - enthalten ist.

## Claims

1. An ether lactone of general structure (I)

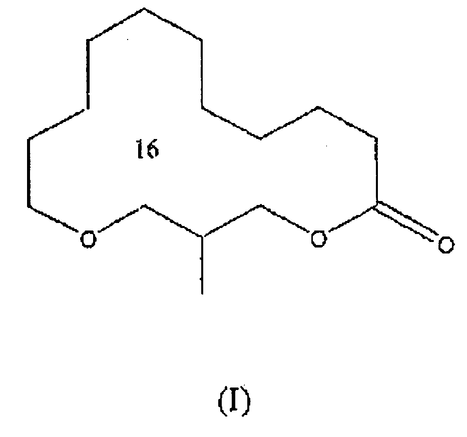

2. A use of said ether lactone of general structure (I) 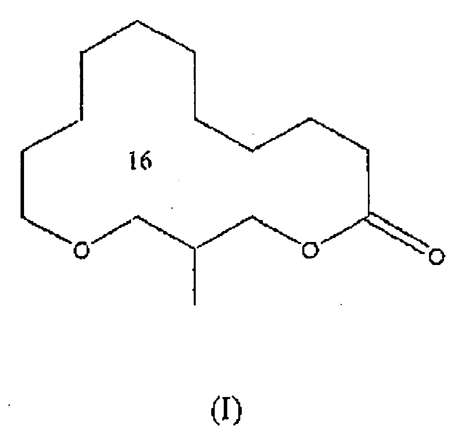 as a fragrance.

3. Fragrance compositions having a content of said ether lactone (I) 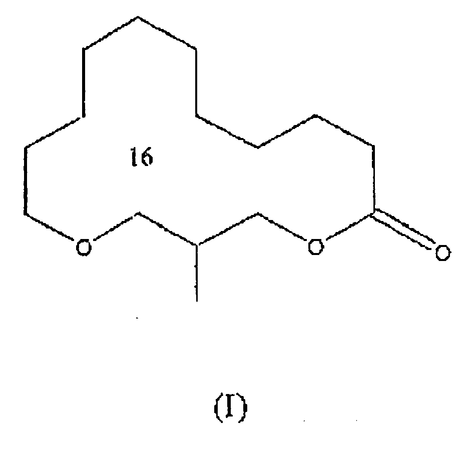 said compound (I) being present in an amount of 1 to 70 % by weight, based on the entire composition.

## Revendications

1. Ether lactone présentant la structure générale (I)

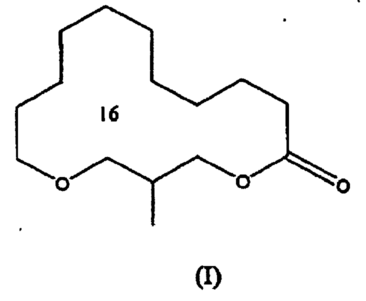

2. Utilisation de l'éther lactone présentant la structure générale (I) 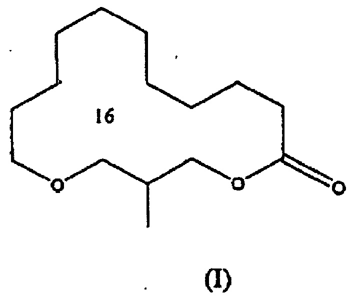 comme matière odorante.

3. Compositions de matière odorante contenant l'éther lactone (I) 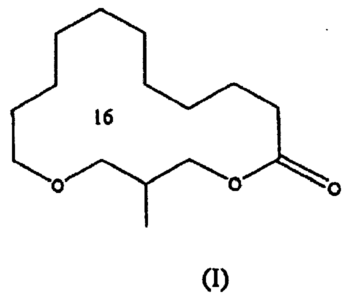 le composé (I) étant contenu à raison de 1 à 70 % en poids par rapport à la totalité de la composition.
